# EUROPEAN PATENT APPLICATION

(11) **EP 4 714 440 A2**
(43) Date of publication of application: **25.03.2026**
(21) Application number: 25199954.6
(22) Date of filing: 03.09.2025
(51) Int. Cl.: A61K 31/353, A61K 36/28, A61K 31/12, A61K 31/185, A61K 33/06, A61K 36/9066, A61K 45/06, A61P 7/02, A61P 7/10, A61P 29/00

(54) **A HEALING HERBAL-MINERAL COMPOSITION WITH ENHANCED EFFECTS**

(30) Priority: 03.09.2024 PL 44966924
(71) Applicant: Kurek, Janusz, 33-112 Tarnowiec (PL)
(72) Inventor: Kurek, Janusz, 33-112 Tarnowiec (PL)
(74) Representative: Godlewski, Piotr

(57) **Abstract**

The subject of the invention is a herbal and mineral composition for the treatment of inflammation, with analgesic, anti-oedema, astringent, anticoagulant and anti-clotting properties, for external application to the patient's skin, containing aluminium salts, calendula extract and arnica extract, wherein arnica contains sesquiterpene lactones and the flavonoid fraction of arnica, additionally containing substances that delay clot formation and/or substances that dissolve existing clots, selected from substances with anti-aggregating, anticoagulant, fibrinolytic, thrombolytic and substances that reduce the number of coagulation factors. The subject of the invention is also the use of the described composition for treating inflammation, pain and swelling.

## Description

The subject of the invention is a medicinal herbal-mineral composition intended for external use in the treatment of inflammation of the skin and subcutaneous tissue (including after insect bites), subcutaneous ecchymosis, bruising, mechanical injuries, first-degree burns, muscle strains and oedema of various origins.

Every day, the human body is exposed to a number of minor or major events that may result in injury. Human skin, as well as muscles and joints, are vulnerable to such injuries. Physical activity, although in itself an important factor for human health, also promotes injuries, including contusions, swellings or bruises.

Polish patent description PL 220 414 describes a medicinal herbal-mineral composition for the treatment of inflammation, with astringent, analgesic and anti-oedema properties, containing aluminium salts, menthol and a mixture of herbs consisting of calendula herb or flower, as well as arnica herb or flower containing sesquiterpene lactones and the flavonoid fraction of arnica.

European patent application EP2364714 describes a composition for use on the skin, in particular for the relief of pain in limbs, tendons and muscles and for the relief of pain in rheumatic diseases, in particular in soft tissue rheumatic diseases, containing at least the following ingredients: carrier, 0.1 to 1.0% by weight of menthol, 1 to 5% by weight of horse chestnut seed extract, 1 to 5% by weight of white willow bark extract, 2 to 8% by weight of arnica tincture, 0.25 to 5% by weight of rosemary oil and 1 to 5% by weight of St. John's wort oil. The subject of the application is also the use of the composition for the manufacture of a medicinal product intended in particular for the external treatment of pain in the limbs, tendons, muscles and rheumatic diseases, in particular rheumatic diseases of soft tissues in humans and animals.

The EP 1834627 patent description discloses a topical composition for application to the skin to inhibit skin irritation, containing one or more anaesthetic ingredients in an effective amount to inhibit the sensation of skin irritation, one or more analgesic ingredients in an effective amount to inhibit the sensation of skin irritation, one or more cooling ingredients in an effective amount to provide a cooling sensation to the skin, and one or more antimicrobial ingredients in an effective amount to inhibit microorganisms on the skin, wherein the analgesic ingredient is selected from the group consisting of acetaminophen, non-steroidal anti-inflammatory drugs (NSAIDs), narcotic drugs, opioid analgesics, drugs used to treat neuropathic pain syndromes, and mixtures thereof.

Polish patent application No. P.356878 describes a synergistic herbal composition consisting of at least two plants containing active substances in the form of iridoids, polyphenols, essential oils, catechin tannins and polysaccharides used in complementary amounts to obtain 1.0 to 1.5% iridoids, 1.5 to 2.0% polyphenols, 0.5-3% catechin tannins and 0.5 to 1.5% polysaccharides per 100 g of the mixture.

The state of the art also includes compositions (usually in the form of ointments or gels) for tired, sore and swollen legs, which contain heparin as an ingredient, as well as compositions intended for local supportive treatment of superficial vein diseases and oedema, contusions or haematomas, whose active substance is heparin.

There are also known compositions indicated for the treatment of ailments associated with vein diseases, e.g. venous thrombosis or superficial vein inflammation, as well as for use on haematomas and bruises on the skin, and swelling resulting from injuries, where the active substance is: polysulphate mucopolysaccharide (heparinoid).

The compositions existing in the prior art have analgesic, anti-edematous, anti-inflammatory and astringent effects. In the treatment of injuries to date, it is known to use aluminium salts with astringent and anti-swelling properties combined with plant extracts with anti-inflammatory and anti-swelling properties, causing simultaneous absorption of the haematoma resulting from the injury. The plant extracts used are mainly arnica extracts (similarly, e.g. great oman), containing helenalin from the sesquiterpene lactone group with anti-inflammatory effects and additionally chelating iron ions found in the blood outside the vascular bed and simultaneously causing their absorption.

Despite the existence of compositions with analgesic, anti-edema and anti-inflammatory properties, as well as compositions currently available on the market, e.g. with heparin (whose composition, however, does not mainly consist of natural plant-based ingredients), there is a need to work on a new composition that would combine the effects of natural ingredients (analgesic, anti-swelling, astringent and anti-inflammatory) with substances that **delay** clot formation (i.e. substances that slow down, hinder or prevent blood clotting), which is important for the course of therapy when the preparation is used immediately after injury, and substances that **dissolve** existing clots, which is important when the preparation is used with a certain delay (some time after the injury, e.g. one or two days).

This effect is achieved by substances with anti-aggregating, anticoagulant, fibrinolytic, thrombolytic and clotting factor-reducing properties and all other substances with a similar effect, as well as substances of natural origin not used in medicine with a similar effect.

The aim of the present invention is therefore to supplement the prior art compositions based on natural ingredients with substances that delay clot formation and dissolve an existing clot.

The subject of the invention is a erbal and mineral composition for the treatment of inflammation, with analgesic, anti-swelling and astringent properties for external application to the patient's skin, containing aluminum salts, calendula extract and arnica extract, wherein arnica contains sesquiterpene lactones and arnica flavonoid fraction, whereas additionally contains substances that delay clot formation and/or substances that dissolve existing clots.

Preferably substances that delay clot formation and dissolve existing clots are selected from among substances with anti-aggregating, anticoagulant, fibrinolytic and thrombolytic properties, as well as substances that reduce the number of deactivating coagulation factors.

Preferably aluminum salts are present in an amount of 0.2% to 20% by weight of the composition, calendula extract is present in an amount of 0.1% to 20% by weight of the composition, arnica extract is present in an amount of 0.1% to 20% by weight of the composition, and substances that delay clot formation and/or dissolve clots are present in an amount of 0.01% to 50% by weight of the composition.

Preferably the solvent is selected from the group consisting of: water, alcohols, fats, glycols, liquid hydrocarbons and ethers.

Preferably the extract is a liquid extract, a thick extract, an extract after partial evaporation of the solvent or a dry extract.

Preferably calendula raw material intended for extraction is fresh or dried raw material in the form of the whole herb, the flower itself or ligulate flowers; the raw material of arnica intended for extraction is the whole herb, arnica flower, the arnica root, or arnica tincture made from fresh or dried flowers, the whole herb or arnica root, whereby the calendula and arnica extracts are selected from a group of extracts obtained using polar, non-polar and supercritical solvents.

Preferably the ratio of raw material to solvent is from 1:5 to 1:10.

Preferably the sesquiterpene lactones include helenalin, dihydrohelenalin and their derivatives, while the flavonoid fraction of arnica contains isoquercitrin, kaempferol and astragalin.

Preferably, the anti-aggregating substance, anticoagulant substance, fibrinolytic substance and thrombolytic substance are selected from, among others, the substances described below.

Preferably the composition may be in the form of a gel, ointment, lotion, cream, emulsion, solution, aerosol, and may also be applied to a patch.

Another subject of the invention is a herbal-mineral composition defined as above for use in the treatment of inflammation, pain and swelling.

**Antiaggregant** substances include (+)-catechin, (-)-epicatechin, (-)-epigallocatechin gallate, (-)-N-(1'-deoxy-1'-D-fructopyranosyl)-S-allyl-L-cysteine, 1,7-bis(4-hydroxy-3-methoxyphenyl)-1,6-heptadiene-3,5-dione, 1-(methyl-sulfonyl)-propylmethyl disulfide, 2,6-dimethoxyphenol, 3,4-dihydroxyacetophenone, 3,4-dihydroxybenzoic acid, 3,5,4'-trihydroxy-6,7-methylenedioxy-3,O-beta-D-glucopyranoside, ,3-alpha,15-dihydroxy-lambda-8(17)-13E-diene, 3-alpha-hydroxy-12,13E-biformene, 3-alpha-hydroxy mannol, 3-beta-hydroxypartenolide, acetyleugenol, adenosine, ajmalicin, ajoene, alginates, allicin, alliin, allylmethyl trisulphide, allyl sulphide, allyl trisulphide, alpha-linolenic acid, anisodamine, anthocyanoside, apigenin, aristolochic acid, ascorbic acid, auraptene, avicine, baicalein, berbamine, berberine, bergapten, bishydroxycoumarin, bromelain, caffeic acid, canin, capsaicin, catechin, cefarantine, cheleritrin, chrysin, cinchonine, cinnamaldehyde, coclaurine, coniferyl alcohol, coumarin, cryptolepin, cryptotanshinone, curcumin, cycloalin, dauricin, dihydrotanshinone-I, elemycin, emodine, epicatechin, estragole, eugenin, eugenol, eugenol acetate, falcarindiol, fangkinoline, ferruginol, ferulic acid, flavanone, flavone, forskolin, frangulin B, gamma-linolenic acid, genistein, gingerol, ginkgolide, ginkgolide-A, ginkgolide-B, ginkgolide-C, ginkgolide, ginsenoside-R-O, ginsenoside-RG-1, ginsenoside RG-2, ginsenosides, heparin, hispidulin, honokiol, imperatorin, isobavachalcone, isoeugenol, isoliquiritigenin, kemferol, kavainin, magnesin, melatonin, melilotin, menthol, methylalloyl trisulphide, methyl isoeugenol, methyl tanshinone, moupinamide, myristicin, nodakenetin, nodakenin, nordihydroguaiaretic acid, oroxylin-A, paeoniflorin, paeonol, paeonolide, panaxynol, parthenolide, phytic acid, pilocarpine, polidatin, protocatechic aldehyde, protopine, puerarin, pyridoxine, quercetin, raubazine, resveratrol, rhynchophyllin, rosmarinic acid, rutin, rutoside, safrole, saikosaponin-A, salicin, salicylates, selenium, serotonin, sodium phenolate, tanshinone, tanshinomn-IIA, tetramethylpyrazine, tetrandrine, thiosulfonate, thymol, tocopherol, timosaponin-A-III, timosaponin-B-I, timosaponin-B-II, timosaponin-B-III, triolein, tyramine, vogonin, Z-guggulsterone, Vitamin E, (-)-acetoxycoline, 14-acetoxycedrol, 2-vinyl-4H-1,3-dithiine, 4-cinnamoylmussatioside, 4-dimethylcaffeoylmussatioside, 6-acetonyldihydronitidine, 6-0-angeloipenoline, acetoxyauraptene, acetoxycoline, anemarrhenasaponin-I, anemarrhenasaponin-IA, anisyl alcohol, artecanin, artocarpanone, artocarpetine, aurantiobtusin, aurantiobtusin-beta-D-glucoside, baicalein, berberastine, broussoauron-A, broussochalcon, broussochalcon-A-tróioctane, broussoflavolone-A, broussoflavolone-E, broussoflavolone-F, budmunchiamine, cepaene, chalepin, chrysoobtusin, chrysoobtusin-glucoside, collinin, cyclanin, danshexinkun-A, decurozide-III, detabilazine, dictamnin, diozmoside, diznecjonyl-cis-kelactone, epoxycoline, futoxin, glucoaurantiobtusin, glucochrysobtusin, hancynon, homoaromoline, huajiaosimulin, hypolaetin-8-glucoside, isotansionon-IIB, istrylobin, cadsuranin A, cadsuranin B, cadsuranin D, cadsuranin E, cadsuranin F, cadsurenone, azarinine, ligustrazine, menthone, N-P-coumaroyltyramine, N-trans-ferulolyticramine, neobavisoflavone, paeonoside, pseudosemiglabrin, paeonoside, pseudosemiglabrin, psychotridine, skullcapflavone-II, watamidine, watamine, vetiver, vetiver-A, valquinine, among others.

**Anticoagulant** substances include (+)-catechin, acetylsalicylic acid, aspirin, acetylsalicylic acid (unnatural), beta-azaron, calophyllolide, citric acid, D-(+)-beta-3,4-dihydrophenyl-lactic acid, D-catechin, dicumarol, ferruginol, heraklenin, hirudin, hirustazin, gilanthin, lapachol, leukoanthocyanidins, p-hydroxycinnamoylferuloylmethane, papain, paeolniflorin, plumbagin, protocatechuic aldehyde, rosmarinic acid, salvianolic acid A, tanshinone II A, hirudinine, dihydroscopoletin, PP'-dihydroxydicinamoylmethane, among others.

**Fibrinolytic** substances include bromelain, curcumin, cycloallin, ginsenoside RO, hementin, niacin, panaxinol, streptokinase, lucioside-N, lucioside-P, among others.

**Thrombolytic** substances include aristolochic acid, aristolochic acid I, aristolochic acid II, chrysin, dicoumarol, ferulic acid, heparin, higenamine, hirudin, oroxylin A, resveratrol, vogonin, sodium ferulan, hirudinin, among others.

Substances are understood to include a single substance, a chemical derivative thereof, as well as a mixture of substances. The examples of substances given are not exhaustive, and the terms anti-aggregating, anticoagulant, fibrinolytic and thrombolytic substances are understood, in accordance with the invention, to include, in addition to those indicated above, any substances exhibiting such effects.

The essence of the action of the indicated substances in the composition is to ensure, when applied topically, a liquid state of 'spilled' blood outside the vascular bed immediately after injury (as it does not disturb the systemic parameters of blood coagulation and allows the damaged vessel to be plugged), as well as after a clot has formed, because thanks to this (liquid state of aggregation) - the blood is 'more accessible' to the action of sesquiterpene lactones: helenalin, dihydrohelenalin and their derivatives, as well as flavonoids (isoquercitrin, kaempferol and astragalin). Thus, the entire healing process and the patient's return to their pre-injury condition is much faster.

The herbal active ingredients combined with aluminium salt have been shown to have an enhanced healing effect leading to a rapid healing of inflammation, petechiae and swelling. A medicinal herbal-mineral composition for the treatment of inflammation, with astringent, analgesic and anti-oedema effects, contains aluminium salts in an amount of from 0.01% to 99.00%, preferably 0.2% to 20%, and a mixture of herbs consisting of calendula in a quantity of 0.001% to 99.000%, preferably 0.1% to 20%, and arnica (containing sesquiterpene lactones and a flavonoid fraction), in a quantity of 0.001% to 99.000%, preferably 0.1% to 20% and 0.01% to 50% depending on the substance used, in all mechanisms affecting blood rheology and dissolution of formed clots, i.e. anti-aggregation, anticoagulation, fibrinolytic and thrombolytic substances. The % composition indicated above refers to weight percentages.

The technical problem solved by the present invention, therefore, consists in providing an easily applied, externally and topically used composition with specialised anti-inflammatory, astringent, analgesic and anti-oedema properties, alleviating the symptoms of skin inflammation, bruises, injuries and burns, based on natural (herbal and mineral) ingredients, which allows the blood outside the vascular bed to remain in a liquid state immediately after injury, as well as after clot formation, as this (fluid state of aggregation) - the blood is 'more accessible' to the action of sesquiterpene lactones: helenalin, dihydrohelenalin and their derivatives, as well as flavonoids (isoquercitrin, kaempferol and astragalin). There is a risk that blood extravasated after injury may penetrate the inter-articular space and disrupt the properties of the synovial fluid. This may impair the proper functioning of the joint. Thanks to the action of the substances mentioned, joints close to the injury can be protected. If clots develop in the inter-articular space, mechanical damage to the cartilage and extensive inflammation can occur. Consequently, there will be an impairment of joint function, combined with pain and swelling. Considering the cartilage reconstruction time, which can take from six weeks to three months (for simple meniscus injuries) or 9-18 months for articular cartilage injuries, the benefit of using the composition according to the invention is invaluable.

Calendula comes in the form of an extract. The term extract is synonymous with the word extract. The raw material of calendula can be the whole herb or the flower itself. The flowers can be harvested whole and processed, or only the so-called ligulate flowers can be harvested. The raw material to be extracted can be fresh or dried. The ratio of raw material to solvent can vary, the standard being 1:5.

The solvent is water, alcohols, fats, glycols, liquid hydrocarbons, ethers. Extracts are obtained by maceration or percolation. Extracts may be liquid, thick, after partial evaporation of the solvent, or dry. Liquid calendula extracts contain up to 30% solvent. Thick calendula extracts contain up to 20% solvent. Dry calendula extracts contain approximately 3% solvent.

Calendula may also be present in the composition in the form of essential oil. Calendula oil is rich in cadinol, alpha-ionone and beta-ionone.

Arnica is also available in extract form. The arnica raw material in the composition according to the invention is a glycol or aqueous extract, or a methanol, ethanol, propanol, oil, glycerine or hydrocarbon extract. The arnica raw material in the composition can be the whole herb, arnica flower or arnica root, but also an arnica tincture made from fresh or dried flowers, whole herb or arnica root, by maceration or percolation, whereby the ratio of raw material to solvent can vary; standard is 1:5, 1:10. Extracts can be concentrated or even dried out completely before being added to the preparation. The raw materials come from any species of Arnica in the genus Arnica (family *Asteraceae = Compositae).*

It is possible to use isolated sesquiterpene lactones of arnica, such as helenalin, dihydrohelenalin and their derivatives, as well as the flavonoid fraction of arnica containing isoquercitrin, kaempferol and astragalin, and to use arnica essential oil as a raw material.

As an aluminum salt, aluminum acetate tartrate or aluminum acetate, or aluminum lactate, or hydrated potassium aluminum sulfate, or anhydrous potassium aluminum sulfate, aqueous aluminum sulfate, or sodium aluminum sulfate, or ammonium aluminum sulfate, or aluminum acetate solution - Burow's water, or aluminum tannin borate - a mixture of aluminum salts with boric and tannic acid in solution, or aluminum tannin acetate - a solution of basic aluminum acetate with tannic acid, or mixtures of water-soluble aluminum compounds with organic acids: malic, tartaric, succinic, malonic, maleic, oxalic, acetic, fumaric, citric, ascorbic, azelaic, adipic, glutaric, picolinic, gluconic, lactic.

Antiaggregants are substances that act by inhibiting the activation and pathological aggregation of platelets, preventing the formation of life-threatening blood clots and embolisms.

Anticoagulant substances, also known as antithrombotic substances, are compounds whose action is based on inhibiting or slowing down the clotting process, i.e. blood loss resulting from tissue damage and vascular disruption.

Fibrinolytic substances, i.e. those that take part in the process of dissolving the clot, which is part of homeostasis and occurs as a result of the activation of a number of enzymes.

Thrombolytic agents are substances used to dissolve intravascular blood clots in order to clear blood vessels.

The base used in the composition may be water, alcohol, PEG-40 hydrogenated castor oil, modified acrylic polymer (Acrylates/C10-30 Alkyl Acrylate Crosspolymer) and any other pharmaceutically and cosmetically acceptable base, allowing the composition to be in the form of an ointment, gel, emulsion, cream and emulgel.

The composition is intended for external use in the form of a gel for skin lubrication. Other forms are also possible, such as milk, cream, ointment, emulsion, solution, spray. The preparation can also be applied to patches.

The composition has specialised anti-inflammatory, astringent, analgesic and anti-oedematous effects and has applications in relieving symptoms of skin inflammation, bruises, injuries and burns. It accelerates the absorption of subcutaneous haemorrhages, post-traumatic effusions, petechiae and oedema, making it an excellent preparation for home treatment of sports injuries, muscle strains after physical exertion, relief of symptoms of sprains and dislocations, inflammation of the subcutaneous tissue around the musculoskeletal system. It relieves pain symptoms in neuralgia and myalgia and is helpful in insect bites. It alleviates the effects of stings from bees and wasps, for example. It improves blood circulation in the lower limbs.

The composition according to the invention is presented in the following examples of implementation, which are not, however, limiting in nature.

### Examples of compositions with anti-aggregant substances

### Example 1

The herbal-mineral composition contains 0.5g aluminum acetate, 1g ethanolic liquid extract of calendula flower (1 part raw material per 5 parts solvent), 1g ethanolic liquid extract of arnica flower (1 part raw material per 5 parts solvent), 0.7g of apigenin, 0.05g of cinnamaldehyde, 0.2g of baicalein, 0.1g of campferol, 0.1g of gingerol, 0.05g of estragole, 2g of tocopherol and a polyacrylic gel base to 100g.

The composition is applied to areas of physical trauma and injury, where it improves local blood circulation, reduces pain and itching, inhibits inflammation in the skin and subcutaneous tissues, reduces swelling and prevents blood cell aggregation, thereby inhibiting the formation of blood clots and thus the resorption of inflammatory exudates and the formation of haematomas, which protects any joints within the injury from the adverse effects of extravasated blood. The composition also has an antiseptic and astringent effect.

The composition prepared according to the aforementioned recipe was applied to a patient with a recent ankle joint injury (sprain). Increasing swelling and the first symptoms of an emerging haematoma were observed. Two hours after the injury, the above-mentioned composition was applied topically in an amount of 5-7 grams. After about 3-4 hours, the application was repeated in a similar manner. No increase in swelling or haematoma was observed. After another 8 hours, the application was repeated, but at this point there was no swelling and no symptoms of hematoma. This was surprising, because such an effect had previously been observed after approximately 3-4 days when using the agents available in the state of the art.

### Example 2

The product in the form of a herbal-mineral composition containing aluminum acetate tartrate in the amount of 1g, ethanolic liquid extract of marigold flower in the amount of 1g (obtained from 1 part of herbal raw material to 5 parts of solvent), ethanolic liquid extract of arnica flower in the amount of 1g (obtained from 1 part by weight of herbal raw material to 5 parts of solvent), alpha-linolenic acid in the amount of 10g and gamma-linolenic acid in the amount of 10g and a base supplemented to 100g with the composition (water, alcohol, hydroxyethylcellulose, polysorbate 80, citric acid) is applied to the place of injury and contusion.

The composition improves local blood circulation, reduces pain and pruritus, inhibits inflammation in the skin and subcutaneous tissues, reduces oedema, seals and strengthens blood vessels, prevents blood cell aggregation and thrombus formation, accelerates resorption of inflammatory exudates and resolution of haematomas, enhances regeneration of the epidermis and connective tissue and, at the same time, has an antiseptic and astringent effect.

The preparation was used on a person after a thigh contusion. It was immediately applied to the bruised area, resulting in no bruising which was an unexpected outcome.

### Example 3

The herbal-mineral composition contains 8 g of aluminum lactate, 0.5 g of aqueous-alcoholic liquid extract from calendula flowers (1 part raw material to 5 parts solvent), 0.5 g of aqueous-alcoholic liquid extract from arnica flowers (1 part raw material to 5 parts solvent), 1 g of aqueous-alcoholic liquid extract from common chamomile flowers (1 part raw material to 5 parts solvent), 2 g of aqueous-alcoholic liquid extract from Baikal skullcap (1 part raw material to 5 parts solvent), 2 g of aqueous-alcoholic liquid extract from Indian nettle (1 part raw material to 5 parts solvent) and a gel base supplemented to 100 g, consisting of hydroxycellulose, glycerol, propyl hydroxybenzoate, alcohol and water.

The preparation was applied to the site of a bruised arm sustained by a person after falling on ice. The large area was severely reddened with signs of emerging swelling. After applying the composition several times at intervals of 2-3 hours, no bruising or swelling was observed the following day.

### Example 4

The herbal-mineral composition contains 2g hydrated potassium aluminum sulphate, 1g liquid extract of calendula flower (1 part raw material per 5 parts solvent), 0.3g liquid glycol extract of arnica flower (1 part raw material per 5 parts solvent), ) 0.5g of water-alcohol liquid extract of oman flower (1 part raw material per 5 parts of solvent), 5g of water-alcohol liquid extract of horse chestnut bark and a gel base up to 100g consisting of; polyethylglycol 40, modified acrylic polymer (Acrylates/C10-30 Alkyl Acrylate Crosspolymer), potassium hydroxide, benzyl alcohol.

### Examples of compositions with anticoagulant substances

### Example 5

The preparation, in the form of a herbal-mineral composition, contains 3g of aluminum tannin-acetate, 3g of oil extract of calendula flower (extract obtained from dry flowers of calendula macerated in cold-pressed sunflower oil in proportions 1:5), 3g of glycol liquid extract of arnica flower (1 part raw material to 5 parts solvent), 0.05g of beta azarone, 0.5g of rosmarinic acid, 2g of citric acid and a base to 100g (water, mineral oil, glycerine stearate, glycerine palmitate, petroleum jelly, glycerine, C12-15pareth 20, cereazine, cetyl alcohol, stearin).

The preparation was applied after an injury resulting from physical exertion (running injury with symptoms of a sprain). In addition to pain, redness and the first symptoms of swelling appeared. After applying the composition as an ointment (about an hour after the injury and at intervals of 2-3 hours thereafter), no haematoma formed and the pain completely subsided. In such situations, haematoma and swelling are standard, hence the result obtained should be considered surprising.

### Example 6

The herbal-mineral composition contains 2g of aluminum tannin-acetate, 0.5g of supercritical CO₂ extract of calendula flower, 0.5g of supercritical CO₂ extract of arnica flower, 0.3g of supercritical CO₂ extract of elecampane flower, 3g of rosmarinic acid and a base to 100g (water, mineral oil, emu oil, caprylic/capric triglycerides, glycerin, acrylates/acrylamide copolymer, mineral oil, polysorbate 85, cetearyl alcohol).

### Example 7

The herbal-mineral composition contains 5g hydrated potassium aluminum sulphate, 1g liquid extract of calendula flower (1 part raw material per 5 parts solvent), 1g supercritical CO₂ extract of arnica flower, 2g water-alcohol liquid extract of rosemary flower (1 part raw material to 5 parts solvent), 0.3g essential oil of cypress, 0.5g essential oil of calamus and a base to 100g (water, mineral oil, emu oil, tricaprylic/capric glycerides, glycerine, acrylates/acrylamide copolymer, mineral oil, polysorbate 85, cetearyl alcohol).

### Example 8

The herbal-mineral composition contains 3g aluminum tannin-acetate, 1g liquid extract of calendula flower (1 part raw material per 5 parts solvent), 2g liquid extract of arnica flower (1 part raw material per 5 parts solvent), 5g aqueous-alcohol liquid extract of rosemary flower (1 part raw material per 5 parts solvent) and a base to 100g (water, mineral oil, emu oil, caprylic/capric triglyceride, glycerine, acrylates/acrylamide copolymer, mineral oil, polysorbate 85, cetearyl alcohol).

### Examples of compositions with thrombolytic substances

### Example 9

The herbal-mineral composition contains 1g of aluminum acetate tartrate, 1g of calendula flower liquid extract (1 part of raw material to 5 parts of solvent), 2g of arnica flower liquid extract (1 part of raw material to 5 parts of solvent), 0.2g of dicoumarol, 0.5g of diosgenin, 0.5g of ferulic acid and a base to 100g (water, mineral oil, white petrolatum, beheneth-10 (behenyl alcohol ethoxylated with 10 moles of ethylene oxide), cetyl alcohol).

The composition, in the form of an ointment, was applied to the injury that had occurred the day before. It manifested as a bruise on the calf and swelling. After applying the ointment to the swollen and bruised area several times during the day, the symptoms diminished the following day. Treatment continued and three days after the injury there were no signs of injury.

### Example 10

The herbal-mineral composition contains 10g of potassium aluminum sulphate dihydrate, 1g of liquid extract of calendula flower (1 part raw material to 5 parts solvent), 2g of liquid extract of arnica flower (1 part raw material to 5 parts solvent), 2g of coumarin and a base to 100g (water, alcohol, hydroxycellulose, polysorbate 80).

### Example 11

The herbal-mineral composition contains 5g of potassium aluminum sulfate dodecahydrate, 1g of calendula flower liquid extract (1 part of raw material to 5 parts of solvent), 1g of arnica flower liquid extract (1 part of raw material to 5 parts of solvent), 1g of melilot hydroalcoholic extract (1 part of raw material to 5 parts of solvent), 1g of yam hydroalcoholic extract, 2g of cattail hydroalcoholic extract (1 part of raw material to 5 parts of solvent), 2g of rosemary flower hydroalcoholic liquid extract (1 part of raw material to 5 parts of solvent) and a water-based base of up to 100 g, containing mineral oil, caprylic/capric triglyceride, glycerine, polyglyceryl-3-methylglucose, glycerol polymethacrylate (and) propylene glycol (and) PVM/MA/carbomer copolymer).

### Example 12

The herbal-mineral composition contains 5g of potassium aluminum sulfate dodecahydrate, 1g of calendula flower liquid extract (1 part of raw material to 5 parts of solvent), 1g of arnica flower liquid extract (1 part of raw material to 5 parts of solvent), 5g of melilot hydroalcoholic extract (1 part of raw material to 5 parts of solvent) and a base to 100g of polyacrylic gel (water, alcohol, hydroxyethylcellulose, polysorbate 80, citric acid).

### Examples of compositions with fibrinolytic substances

### Example 13

The preparation is a herbal-mineral composition containing 1g of aluminum acetate tartrate, 1g of ethanolic calendula flower liquid extract (1 part of raw material to 5 parts of solvent), 1g of ethanolic arnica flower liquid extract (from 1 part by weight of raw material to 5 parts of solvent), 2g of curcumin, 3g of niacin, 1g of salicylic acid, 0.5g of streptokinase and a base to 100g (water, mineral oil, caprylic/capric triglyceride, glycerin, polyglyceryl-3-methylglucose, glyceryl polymethacrylate (and) propylene glycol (and) PVM/MA/ copolymer, carbomer).

The preparation in the form of an ointment was applied to a fresh injury on the back of the hand following a strong blow, which damaged a blood vessel, resulting in significant bruising visible immediately after the injury. A thicker layer of ointment was applied to the affected area, repeating the application every few hours. No clot formation was observed, and after the clotted blood was absorbed, there was no trace left.

### Example 14

The herbal-mineral composition contains 0.5g of aluminium acetate, 1g of calendula flower liquid extract (1 part raw material to 5 parts solvent), 1g of arnica flower liquid extract (1 part raw material to 5 parts solvent, 3g of curcumin and a polyacrylate gel base to 100g, applied to a patch.

### Example 15

The herbal-mineral composition contains 1.5g of aluminium acetate tartrate, 1g of calendula flower liquid extract (1 part of raw material to 5 parts of solvent), 1g of arnica flower liquid extract (1 part of raw material to 5 parts of solvent), 1g of turmeric rhizome alcoholic extract, 1g of meadowsweet hydroalcoholic extract (1 part of raw material to 5 parts of solvent), 3g of Tamanu oil (Calophyllum Inophyllum) and a 100g vehicle based on water, mineral oil, caprylic/capric triglyceride, glycerin, polyglyceryl-3-methylglucose, glyceryl polymethacrylate (and) propylene glycol (and) PVM/MA/ copolymer, carbomer.

### Example 16

The herbal-mineral composition contains 1.5g of aluminium acetate tartrate, 1g of calendula flower liquid extract (1 part of raw material to 5 parts of solvent), 1g of arnica flower liquid extract (1 part of raw material to 5 parts of solvent), 3g of hirudin and a vehicle up to 100g based on water, mineral oil, caprylic/capric triglyceride, glycerin, polyglyceryl-3-methylglucose, glyceryl polymethacrylate (and) propylene glycol (and) PVM/MA/ copolymer, carbomer.

### Example of a composition with anti-coagulation agents

### Example 17

The herbal-mineral composition contains 1.5g of aluminum acetate tartrate, 1g of calendula flower liquid extract (1 part of raw material to 5 parts of solvent), 1g of arnica flower liquid extract (1 part of raw material to 5 parts of solvent), 0.5g of heparin and a base to 100g based on water, mineral oil, caprylic/capric triglyceride, glycerin, polyglyceryl-3-methylglucose, glyceryl polymethacrylate (and) propylene glycol (and) PVM/MA copolymer, carbomer
The composition has been applied to a patch that is applied to the site of injury and trauma.

### Example 18

A composition was prepared with the following composition, in which the given amounts are % by weight:
Demineralised water (62.01%)
Natrosol (3.4%)
Aluminum acetate tartrate (1%)
Calendula glycolic extract (1%)
Arnica glycolic extract (1%)
Oman (*Inula*) extract (0.5%)
Willow extract (water-alcohol) (1%)
Reed extract (1%)
Turmeric extract (water-alcohol) (1%)
*Ruta graveolens* extract (1%)
Tocopherol (0.5%)
DMSO (5%)
Milkweed extract *(Melilotus officinalis*) (1%)
Polysorbate 80 (0.05%)
Euxyl PE 9010 (0.35%)
Euxyl K 712 (0.25%)
Denatured alcohol (19.24%)
Orange perfume oil (0.35%)
Menthol (0.35%)

The composition as defined above was studied by testing its efficacy on a group of patients with symptoms as shown in Table 1 below:

**Table 1**

| **No .** | **Gender and age of the patient M-male/ K-woman** | **Type of injury** | **Symptoms** | **Method of application** | **Effect achieved** |
|---|---|---|---|---|---|
| 1 | M (27) | Ankle joint injury | Swelling, redness, initial signs of blood extravasation, pain rated at 7 on the VAS scale | Following application of the composition directly to the site of injury, rapid pain relief appeared, rated at 3 after 12 hours | rapid inhibition of haematoma formation |
| 2 | M (25) | Thigh contusion | Pain rated at 6 on the VAS scale, developing swelling, redness | The composition was applied immediately after the injury, pain relief appeared after 1 hour, no haematoma formed. | no bruising |
| 3 | K (38) | Contusion of the arm after a fall on ice | Pain rated at 5 on the VAS scale, redness, slight numbness of the right hand | The composition was applied immediately after the injury, and no haematoma formation was observed after approximately 24 hours | no haematoma, trace swelling |
| 4 | K (23) | Ankle strain during running | Pain rated at 5 on the VAS scale, swelling, redness | First application after one hour, then several times every 2-3 hours; the injury occurred in the afternoon; in the morning, the joint showed no signs of pathology. | No swelling or pain |
| 5 | M (26) | Calf injury due to impact during football training | Pain rated at 5 on the VAS scale, redness, visible haematoma | The ointment was applied on the second day, several times, every 3 to 4 hours, on the following day the haematoma changed colour and shrank, on the following day the remaining slight shading of the haematoma area, the pain and swelling disappeared | Rapid resorption of haematoma, two days after injury |
| 6 | M (29) | Fresh injury to the hand, metacarpus , dorsal part, as a result of a strong blow | Pain rated at 7 on the VAS scale, visible, spreading haematoma, rapidly forming and spreading subcutaneous haemorrhage, as a result of vessel rupture | The ointment was applied within an hour of the injury, in a thick layer, and applied for two days, 3-4 times a day, except at night, when the thick layer of ointment was secured with a dressing | Inhibition of clot formation and rapid resorption of the haematoma |
| 7 | M (21) | Knee joint injury during training, following a collision with another athlete | Increasing pain and swelling, after a few hours significant restriction of joint mobility with a small visible haematoma | Immediately after the injury, local cooling and an ice pack were applied, providing slight relief. After several hours, the composition was applied 4-5 times a day, and results were observed after two days. | Swelling disappeared, joint mobility returned, no pain symptoms |
| 8 | K (24) | Ankle injury, following a fall from a horse during equestrian training | Pain rated at 7 on the VAS scale, increasing swelling and bruising, worsening pain when attempting to move the joint. | The composition was applied approximately 1.5 hours after the injury, with repeated applications every three hours, with a break overnight, when a dressing was applied to protect and stabilise the joint after the ointment was applied. | Relief of swelling, restoration of joint mobility, resorption of haematoma. The following day, the joint regained sufficient mobility for the volunteer to take part in the competition |
| 9 | M (52) | Thigh and hip injury after a fall | Extensive bruising, pain rated at 6 on the VAS scale, swelling | The composition was applied within an hour after the fall, used approximately every 4 hours, lubricating the entire area of the injury | After two days, there was a significant reduction in bruising, pain rated at 3 on the VAS scale, no hip mobility restrictions, resorption of the haematoma |
| 10 | K (58) | Facial bruising, bilateral, combined with swelling, following ENT surgery | Due to the administration of painkillers after the procedure, the level of pain was not determined | A preparation containing aluminium acetate, arnica and calendula was applied to one side of the face, and the above-mentioned composition was applied to the other side. | Reduction in swelling and bruising. After two days of use, there was a clear difference between the sides of the face. Bruising was significantly reduced when using the composition |
| 11 | K (19) | Radiocarpal joint injury during volleyball training | Pain rated at 7 on the VAS scale and increasing swelling | The composition was applied immediately after the injury, approximately every 4 hours. | After about a day of use, the pain had subsided and there was no swelling of the joint |
| 12 | K (26) | Overloading of the shoulder joint as a result of swimming training | Pain rated at 6-7 on the VAS scale, slight swelling, restricted mobility | The symptoms appeared after the training, with increasing pain, after about three hours the composition was applied and continued for three days every 4-5 hours, with a break for the night, when after the application a dressing was applied to protect and stabilize the joint. | Swelling and pain relief. Increased mobility of the joint was observed and on the following day full mobility returned |
| 13 | M (47) | Ocular haematoma resulting from head injury | Significant bruising around the eye sockets, swelling of the eyelids, pain was not assessed due to the administration of painkillers | The composition was applied four times a day for three days | Complete absorption of the haematoma |
| 14 | M (23) | Haematoma due to impact, biceps femoris muscle, after a kick during training | Pain rated at 7 on the VAS scale, haematoma | The composition was applied immediately after the injury. After approximately 24 hours of application every 4-5 hours, slight shading of the haematoma site was visible, and after another 24 hours it also disappeared. | No pain, resorption of haematoma |
| 15 | K (22) | Haematoma in the hip area after a skiing fall | Extensive haematoma in the hip and thigh area, pain on the VAS scale of 5, one day after the fall. | Visible, extensive haematoma, approximately 24 hours after the fall, the composition was applied to the existing haematoma, the composition was applied every 5-6 hours, along with restricted physical activity, with an approximately 8-hour break in application at night | after three days of application the haematoma disappeared, complete resorption of the haematoma |
| 16 | M (39) | Hand injury - short thumb abductor muscle, as a result of an impact during physical work | Rapidly appearing bruising and pain rated at 7 on the VAS scale, two hours after injury | The composition was applied every 4-5 hours, overnight, for approx. 8 hours, the hand was stabilised with an elastic bandage, there was no interruption in tissue continuity. | Resorption of the haematoma. After two days, absorption of the haematoma was observed, there was no swelling or pain, mobility of the hand was full. |

The table above clearly shows that in all cases (patients with different injuries), a reduction in pain, reduction or absence of swelling and bruising, resorption of haematomas and restoration of full function were observed. The observed results on the test group, in comparison with the comparative example below, allow us to conclude that the composition according to the invention definitely speeds up the process of the patient's return to the pre-injury condition. It reduces pain, redness, bruising and swelling, and restores full motor function to the injured area.

### Comparative example of a composition from the prior art without ingredients that delay clot formation and dissolve existing clots, and a composition according to the invention containing such ingredients

### Example 18

Thanks to the composition used, it was possible to accelerate the return to full joint mobility and, above all, to protect the joint from damage caused by the mechanical action of crystallised - precipitated/solidified haemorrhages, which, due to their significant iron content from clotted blood, cause damage to the joint surface.

The commercially available composition and the composition according to the invention were applied in the form of an ointment applied to the ankle joint immediately after the injury. The commercially available composition contained calendula, arnica, aluminium salts and menthol, while the composition according to the invention was the composition described in Example 1.

A comparison of the compositions used (applied twice at intervals of several hours) is presented in Table 2 below:

**Table 2. Comparison of the properties of the composition according to the invention with commercially available**

| | **Commercial composition** | **Composition according to the invention** |
|---|---|---|
| Occurrence of haematoma immediately after the injury and application of the composition | YES | NO |
| Occurrence of swelling immediately after the injury and application of the composition | YES | NO |
| Pain on the VAS scale | 5-6 | 2-3 |
| Discomfort (pain) felt by the patient when the composition is applied 3 times a day to the area of the injured joint | Over 3 days | After 2 days, the patient had no pain. Return to full fitness. |

Expanding the composition of the compositions used so far with new ingredients, especially those that prevent the formation of a clot and the resulting damage/blockage of the joint and the triggering of inflammation that increases swelling and pain, and at the same time those that dissolve the already formed clot, allows for the quick elimination of the consequences of the injury and a quick return to full fitness.

The use of a proprietary composition also eliminates the risk of joint damage by preventing clots from entering the joint space, causing degrading and even permanent damage to the joint cartilage. This situation leads to pain, even after the oedema and haematoma have cleared up, and restricts mobility (including sporting activities) or even makes daily life difficult. The consequence of such injuries, can be osteoarthritis of the joint. Hence, the unique composition of natural ingredients, apart from faster regeneration after the injury itself, also prevents long-term and troublesome conditions, such as the aforementioned osteoarthritis.

## Claims

1. Herbal and mineral composition for the treatment of inflammation, with analgesic, anti-swelling and astringent properties for external application to the patient's skin, containing aluminum salts, calendula extract and arnica extract, wherein arnica contains sesquiterpene lactones and arnica flavonoid fraction, **characterised in that** it additionally contains substances that delay clot formation and/or substances that dissolve existing clots.

2. Composition according to claim 1, **characterised in that** substances that delay clot formation and dissolve existing clots are selected from among substances with anti-aggregating, anticoagulant, fibrinolytic and thrombolytic properties, as well as substances that reduce the number of deactivating coagulation factors.

3. Composition according to any of claims 1 to 2, **characterised in that** aluminum salts are present in an amount of 0.2% to 20% by weight of the composition, calendula extract is present in an amount of 0.1% to 20% by weight of the composition, arnica extract is present in an amount of 0.1% to 20% by weight of the composition, and substances that delay clot formation and/or dissolve clots are present in an amount of 0.01% to 50% by weight of the composition.

4. Composition according to any of the preceding claims, **characterised in that** the solvent is selected from the group consisting of: water, alcohols, fats, glycols, liquid hydrocarbons and ethers.

5. Composition according to claim 3, **characterised in that** the extract is a liquid extract, a thick extract, an extract after partial evaporation of the solvent or a dry extract.

6. Composition according to any of the preceding claims, **characterised in that** the calendula raw material intended for extraction is fresh or dried raw material in the form of the whole herb, the flower itself or ligulate flowers; the raw material of arnica intended for extraction is the whole herb, arnica flower, the arnica root, or arnica tincture made from fresh or dried flowers, the whole herb or arnica root, whereby the calendula and arnica extracts are selected from a group of extracts obtained using polar, non-polar and supercritical solvents.

7. Composition according to any of the claims, **characterised in that** the ratio of raw material to solvent is from 1:5 to 1:10.

8. Composition according to claim 1, **characterised in that** the sesquiterpene lactones include helenalin, dihydrohelenalin and their derivatives, while the flavonoid fraction of arnica contains isoquercitrin, kaempferol and astragalin.

9. Composition according to claim 2, **characterised in that** the anti-aggregating substance is selected from, among others: (+)-catechin, (-)-epicatechin, (-)-epigallocatechin gallate, (-)-N-(1'-deoxy-1'-D-fructopyranosyl)-S-allyl-L-cysteine, 1,7-bis(4-hydroxy-3-methoxyphenyl)-1,6-heptadiene-3,5-dione, 1-(methyl-sulfonyl)-propylmethyl disulfide, 2,6-dimethoxyphenol, 3,4-dihydroxyacetophenone, 3,4-dihydroxybenzoic acid, 3,5,4'-trihydroxy-6,7-methylenedioxy-3,O-beta-D-glucopyranoside, ,3-alpha,15-dihydroxy-lambda-8(17)-13E-diene, 3-alpha-hydroxy-12,13E-biformene, 3-alpha-hydroxy mannol, 3-beta-hydroxypartenolide, acetyleugenol, adenosine, ajmalicin, ajoene, alginates, allicin, alliin, allylmethyl trisulphide, allyl sulphide, allyl trisulphide, alpha-linolenic acid, anisodamine, anthocyanoside, apigenin, aristolochic acid, ascorbic acid, auraptene, avicine, baicalein, berbamine, berberine, bergapten, bishydroxycoumarin, bromelain, caffeic acid, canin, capsaicin, catechin, cefarantine, cheleritrin, chrysin, cinchonine, cinnamaldehyde, coclaurine, coniferyl alcohol, coumarin, cryptolepin, cryptotanshinone, curcumin, cycloalin, dauricin, dihydrotanshinone-I, elemycin, emodine, epicatechin, estragole, eugenin, eugenol, eugenol acetate, falcarindiol, fangkinoline, ferruginol, ferulic acid, flavanone, flavone, forskolin, frangulin B, gamma-linolenic acid, genistein, gingerol, ginkgolide, ginkgolide-A, ginkgolide-B, ginkgolide-C, ginkgolide, ginsenoside-R-O, ginsenoside-RG-1, ginsenoside RG-2, ginsenosides, heparin, hispidulin, honokiol, imperatorin, isobavachalcone, isoeugenol, isoliquiritigenin, kemferol, kavainin, magnesin, melatonin, melilotin, menthol, methylalloyl trisulphide, methyl isoeugenol, methyl tanshinone, moupinamide, myristicin, nodakenetin, nodakenin, nordihydroguaiaretic acid, oroxylin-A, paeoniflorin, paeonol, paeonolide, panaxynol, parthenolide, phytic acid, pilocarpine, polidatin, protocatechic aldehyde, protopine, puerarin, pyridoxine, quercetin, raubazine, resveratrol, rhynchophyllin, rosmarinic acid, rutin, rutoside, safrole, saikosaponin-A, salicin, salicylates, selenium, serotonin, sodium phenolate, tanshinone, tanshinomn-IIA, tetramethylpyrazine, tetrandrine, thiosulfonate, thymol, tocopherol, timosaponin-A-III, timosaponin-B-I, timosaponin-B-II, timosaponin-B-III, triolein, tyramine, vogonin, Z-guggulsterone, Vitamin E, (-)-acetoxycoline, 14-acetoxycedrol, 2-vinyl-4H-1,3-dithiine, 4-cinnamoylmussatioside, 4-dimethylcaffeoylmussatioside, 6-acetonyldihydronitidine, 6-0-angeloipenoline, acetoxyauraptene, acetoxycoline, anemarrhenasaponin-I, anemarrhenasaponin-lA, anisyl alcohol, artecanin, artocarpanone, artocarpetine, aurantiobtusin, aurantiobtusin-beta-D-glucoside, baicalein, berberastine, broussoauron-A, broussochalcon, broussochalcon-A-tróioctane, broussoflavolone-A, broussoflavolone-E, broussoflavolone-F, budmunchiamine, cepaene, chalepin, chrysoobtusin, chrysoobtusin-glucoside, collinin, cyclanin, danshexinkun-A, decurozide-III, detabilazine, dictamnin, diozmoside, diznecjonyl-cis-kelactone, epoxycoline, futoxin, glucoaurantiobtusin, glucochrysobtusin, hancynon, homoaromoline, huajiaosimulin, hypolaetin-8-glucoside, isotansionon-IIB, istrylobin, cadsuranin A, cadsuranin B, cadsuranin D, cadsuranin E, cadsuranin F, cadsurenone, azarinine, ligustrazine, menthone, N-P-coumaroyltyramine, N-trans-ferulolyticramine, neobavisoflavone, paeonoside, pseudosemiglabrin, paeonoside, pseudosemiglabrin, psychotridine, skullcapflavone-II, watamidine, watamine, vetiver, vetiver-A, valquinine, their derivatives or mixtures.

10. Composition according to claim 2, **characterised in that** the anticoagulant is chosen from among others: (+)-catechin, acetylsalicylic acid, aspirin, acetylsalicylic acid (unnatural), beta-azaron, calophyllolide, citric acid, D-(+)-beta-3,4-dihydrophenyl-lactic acid, D-catechin, dicumarol, ferruginol, heraklenin, hirudin, hirustazin, gilanthin, lapachol, leukoanthocyanidins, p-hydroxycinnamoylferuloylmethane, papain, paeolniflorin, plumbagin, protocatechuic aldehyde, rosmarinic acid, salvianolic acid A, tanshinone II A, hirudinine, dihydroscopoletin, PP'-dihydroxydicinamoylmethane, their derivatives or mixtures.

11. Composition according to claim 2, **characterised in that** the fibrinolytic substance is selected from, among others: bromelain, curcumin, cycloallin, ginsenoside RO, hementin, niacin, panaxynol, streptokinase, lucyozide-N, lucyozide-P, their derivatives or mixtures thereof.

12. Composition according to claim 2, **characterised in that** the thrombolytic substance is selected from, among others: aristolochic acid, aristolochic acid I, aristolochic acid II, chrysin, dicumarol, ferulic acid, heparin, hygenamine, hirudin, oroxylin A, resveratrol, vogonin, sodium ferulan, hirudinin, their derivatives or mixtures thereof.

13. Composition according to any of the above claims, **characterised in that** it may be in the form of a gel, ointment, lotion, cream, emulsion, solution, aerosol, and may also be applied to a patch.

14. Composition specified by any of the above claims for use in the treatment of inflammation, pain and swelling.
